(19) Europäisches Patentamt
European Patent Office
Office européen des brevets

(11) **EP 2 781 164 A1**

(12) **EUROPEAN PATENT APPLICATION**
published in accordance with Art. 153(4) EPC

(43) Date of publication:
**24.09.2014 Bulletin 2014/39**

(51) Int Cl.:
***A23J 3/14*** *(2006.01)*

(21) Application number: **12850326.5**

(22) Date of filing: **05.11.2012**

(86) International application number:
**PCT/JP2012/078660**

(87) International publication number:
**WO 2013/073404 (23.05.2013 Gazette 2013/21)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.11.2011 JP 2011249095**

(71) Applicant: **Kameda Seika Co., Ltd.
Niigata 950-0198 (JP)**

(72) Inventors:
• **FUJII, Mikio
Niigata-shi
Niigata 950-0198 (JP)**

• **HIRATSUKA, Takamasa
Niigata-shi
Niigata 950-0198 (JP)**
• **KADOWAKI, Motoni
Niigata-shi
Niigata 950-2181 (JP)**

(74) Representative: **Maiwald Patentanwalts GmbH
Elisenhof
Elisenstrasse 3
80335 München (DE)**

(54) **RICE-PROTEIN COMPOSITION AND METHOD FOR MANUFACTURING SAME**

(57)   The objective of the present invention is to provide a high-purity rice-protein composition with improved tongue feel and a melt-in-the-mouth quality, and a method for manufacturing the composition. The rice-protein composition comprises glutelin and prolamine, the protein content with respect to the solid content being at least 90%. Rice protein extracted by an alkaline solution from rice or rice flour is neutralized and coagulated at a temperature no lower than 40 80 DEG C, or is coagulated by adding a divalent metal ion and then neutralized, or glucono-delta-lactone is added and the solution is heated to gel the protein; by therein keeping the pH of the rice-protein composition no lower than 7.0 or no higher than 4.0, a rice-protein composition with extremely good dispersibility and tongue feel can be obtained. Further, by heating to at least 80 DEG C after adjusting pH, a composition with high water absorbency is achieved.

FIG. 1

EP 2 781 164 A1

**Description**

TECHNICAL FIELD

The present invention relates to a rice-protein composition that is high purity and excels in dispersibility and tongue feel, and a method for manufacturing this rice-protein composition.

BACKGROUND ART

Conventionally, rice protein has a cholesterol lowering action and lipid metabolism improving action, and has been given focus as a raw material of functional foods. It has been disclosed that such rice protein can be extracted by immersing rice or rice flour in an alkaline solution, and neutralizing the extraction liquid to collect as aggregating precipitate (Patent Document 1), and it has been disclosed that the rice-protein composition obtained by this method excels in nutritional value, and also excels in digestion and absorption (Non-Patent Document 1 and Non-Patent Document 2).

Regarding the alkali extraction liquid containing rice protein, the waste liquid upon producing rice starch is disclosed in Non-Patent Document 3, in addition to Patent Document 1. More specifically, the matter of eluting to remove about 50% of the protein in rice grains by immersing polished rice in a sodium hydroxide solution for 2 to 3 days, and preparing stock feed by pH treating and dehydrating after mixing waste liquid from this alkali treatment and other waste matter have been disclosed. This method is carried out with the purpose of removing rice protein, which is a waste matter when collecting rice starch; however, it resembles the process of collecting rice protein in regards to the operations thereof in themselves.

[Patent Document 1] Japanese Unexamined Patent Application, Publication No. 2006-273840
[Non-Patent Document 1] Kumagai et al.; J. Nutr. Sci. Vitaminol., Vol. 52, pp. 467-572 (2006)
[Non-Patent Document 2] Kumagai et al.; J. Nutr. Sci. Vitaminol., Vol. 55, pp. 170-177 (2009)
[Non-Patent Document 3] Saitou, Shouzou, Rice Starch, J. Nikuni (Edited), "Starch Science Handbook," pp. 385-395, Asakura Press (1977)

DISCLOSURE OF THE INVENTION

Problems to be solved by the Invention

However, for rice-protein compositions obtained by the conventional methods, the constitutional particles are hard and pulverizing is difficult, and thus the tongue feel and melt-in-the-mouth quality are inferior with a gritty food texture. For this reason, conventional rice-protein compositions have had the problem in that the applicable foods are limited.

In addition, upon collecting rice protein industrially, it is particularly important to collect in a state in which the rice starch abundantly contained in the raw material rice is high quality from the viewpoint of economy, and it goes without saying that a technique has been demanded for collecting the rice protein occurring as a byproduct of the process of producing rice starch in a high quality state.

The present inventors have already discovered that, by adjusting the temperature upon generating an aggregate of rice protein, the obtained protein composition will display superior tongue feel and melt-in-the-mouth quality (Japanese Patent Application No. 2010-156467), and when introducing divalent metal ion to a rice protein-containing extraction liquid, the rice protein generates an aggregate, and the protein composition obtained by collecting this aggregate displays superior tongue feel and melt-in-the-mouth quality (Japanese Patent Application No. 2010-156466), and thus are applying for patents.

However, further improvements in the tongue feel and melt-in-the-mouth quality of rice-protein compositions has been demanded.

The present invention has been made taking the above actual circumstances into consideration, and has an object of providing a rice-protein composition for which tongue feel and melt-in-the-mouth quality are improved, and a manufacturing method thereof.

Means for Solving the Problems

As a result of thorough investigation of the manufacturing process of rice starch and the characteristics, properties, etc. of rice protein derived therefrom, the present inventors found that a composition containing glutelin and prolamine, which are the principle protein components of rice endosperm, in compositional ratios equivalent to the abundance ratio in rice endosperm, and obtained by gelling rice protein in which the protein content relative to the solids content is at least a predetermined value under fixed conditions, will excel water-retaining capacity, and will be very superior in tongue feel and melt-in-the-mouth quality, thereby arriving at completing the present invention. More specifically, the present invention provides the following matters.

According to a first aspect, a rice-protein composition includes glutelin and prolamine, in which the protein content with respect to the solid content is at least 90%, and can retain water in at least 6 times its weight while maintaining a gelatinous form.

According to a second aspect, a rice-protein composition includes glutelin and prolamine, in which a pH of liquid when dissolved or suspended in water is at least 7.0 or no higher than 4.0.

According to a third aspect, a method for manufacturing a rice-protein composition, includes the steps of: forming an aggregate of protein by neutralizing a solution containing rice protein, or by adding a divalent metal ion to the solution; and gelling to collect the aggregate by heating to at least 80°C.

According to a fourth aspect, in the method for manufacturing a rice-protein composition as described in the third aspect, the pH of a solution containing rice protein is at least 11.3.

According to a fifth aspect, in the method for manufacturing a rice-protein composition as described in the third aspect, the generation of the aggregate is performed at a temperature of at least 40°C and no higher than 80°C.

According to a sixth aspect, in the method for manufacturing a rice-protein composition as described in any one of the third to fifth aspects, the pH of the solution during the heating is set to at least 7.0 or no higher than 4.0.

According to a seventh aspect, the method for manufacturing a rice-protein composition as described in any one of the third to sixth aspects includes a step of passing a solution containing rice protein through a sieve of no more than 75 $\mu$m mesh size.

According to an eighth aspect, the method for manufacturing a rice-protein composition as described in any one of the third to seventh aspects includes a step of concentrating and desalinating rice protein by membrane filtrating an alkaline extraction liquid containing rice protein using a microfiltration membrane or ultrafiltration membrane.

According to a ninth aspect, a method for manufacturing a rice-protein composition includes a step of adjusting a solution containing rice protein to a pH of at least 7.0 or no higher than 4.0, and then drying as is, or collecting a precipitate formed and then drying.

According to a tenth aspect, a method for manufacturing a rice-protein composition includes a step of adding glucono-delta-lactone to a solution containing rice protein in an amount making a final pH of a gel at least 7.0 or no higher than 4.0, and then causing protein to gel by heating to at least 80°C.

Effects of the Invention

According to the present invention, the formation of hard aggregates being suppressed, a high-purity rice-protein composition being obtained, and this composition exhibiting superior tongue feel and melt-in-the-mouth quality.

BRIEF DESCRIPTION OF THE DRAWINGS

FIG. 1 is a photograph showing the existence of glutelin and prolamine in Sample A and Sample B;
FIG. 2 is a photograph showing the influence from the neutralizing temperature and subsequent heating on the behavior of free SH groups of rice-protein samples; and
FIG. 3 is an illustration showing a molecular model proposed as an aggregation mechanism of rice-protein molecules.

PREFERRED MODE FOR CARRYING OUT THE INVENTION

Hereinafter, an embodiment of the present invention will be explained; however, this is not to limit the present invention.

First, a method for manufacturing rice starch of recent years will be outlined. The raw materials of rice starch may be short-grain rice that is Japonica, long-grain rice that is Indica, etc. Usually, polished rice having a degree of milling around 90% is used. The rice is washed in order to remove impurities on the polished rice surface and rice bran component. To the rice on which rice washing has completed, an alkaline solution, for example, a 0.1 to 1.0% by weight sodium hydroxide aqueous solution, is added in 1- to 5-times the amount of rice, immersing for a half day to two days to soften the structure of rice, and this is pulverized. Pulverizing may be performed in a dry process by retrieving only the immersed rice, or may be performed in a wet process while simultaneously adding immersed rice and immersion water, or newly adding sodium hydroxide or tap water. Rice starch disaggregates to single granules from pulverizing, and rice protein dissolves or disperses/suspends in an alkaline solution. Since coarse granules of starch and pulverizing residue, insoluble dietary fiber, etc. are included in the emulsion obtained from the above-mentioned process, these are filtrated with a 150-mesh screen. Rice starch is produced by passing a starch emulsion subjected to filtration through concentration/purification with a continuous-type centrifuge such as a nozzle separator, dehydration, drying and screening.

Rice protein is contained in large amounts in the immersion liquid obtained at the early stage of the above-mentioned process, the centrifuge supernatant of the alkaline pulverization emulsion of rice, for example, light fluid of nozzle separator (hereinafter collectively referred to as "rice protein alkaline extraction liquid"). Normally, these liquids containing rice protein are processed as waste water, and the dried matter obtained through processes of neutralization, dehydration and drying is employed as stock feed. The above-mentioned dried matter has coarse particle which are difficult to finely pulverize due to being hard, and lacks in water absorbency and dispersibility in water; therefore, it has been thus far difficult to use in foodstuffs ingested by humans.

Rice protein exists as granules (protein body) in rice endosperm, mainly, and by alkaline extraction, it is considered that a part dissolves in solution, and the remaining part floats in the liquid while retaining a swollen protein body form. In addition, a large amount of dietary fiber mixes in the rice protein alkaline extraction liquid, and it is considered that insoluble dietary fiber in neutral to weakly acidic liquids and soluble in alkaline liquids are present in this. Upon neutralizing rice protein to cause to aggregate, it is expected that dietary fiber having such a property forms aggregates of rice protein, or huge aggregates in which protein bodies crosslink. Actually, after pretreating rice or rice flour in advance with enzyme preparation containing cellulase, hemicellulase or pectinase, which are a dietary fiber degrading enzymes, the rice-protein composition collected by alkaline extracting protein, and neutralizing the extract has been confirmed to have very superior dispersibility compared to a case of not carrying out enzyme preparation treatment, and the legitimacy of this hypothesis has been confirmed.

As a method of producing rice protein alkaline extraction liquid having low dietary fiber content, in addition to allowing enzyme preparation to act in advance on the rice or rice flour to degrade and remove the dietary fiber, it is effective to filtrate the dietary fiber by sieve processing the obtained rice protein alkaline extraction liquid. In the present invention, in order to efficiently remove dietary fiber, it is necessary to process with a sieve of at least 200 mesh (sieve size of 75 $\mu$m or less) for JIS standard test sieves; however, it is preferably processed with a sieve of at least 280 mesh (mesh size of 53 $\mu$m or less). It is also possible to further use a fine mesh size sieve or filter cloth, whereby at least equivalent effects for removal of dietary fiber are expected. However, since the processing rate of filtration declines as the mesh size becomes smaller, it is desirable to process with a sieve of no more than 300 mesh (mesh size of 50 $\mu$m or more) in industrial operation. It should be noted that, since rice protein alkaline extraction liquid is collected as light fluid from centrifugation by a nozzle separator or the like of an alkaline pulverization emulsion of rice, a slight amount of starch granules may be mixed therein. Since mixing of starch granules causes the protein purity of the obtained rice-protein composition to decline dramatically, it is desirable to process the rice protein alkaline extraction liquid by centrifugation or the like again so as to the utmost remove residual starch. By filtrating dietary fiber, the purity of the collected rice protein will greatly rise, and it becomes possible to obtain a rice-protein composition having at least 90% protein content relative to the solid content, and by performing removal alkali components and low-molecular-weight components other than salts as appropriate, the content of protein relative to the solid content can be made at least 95%.

In addition to the physical methods by centrifugation, etc. for removal of starch, an enzymatic method using amylase can also be employed. Since most amylase has activity for neutral and weakly acidic, it is difficult to make function in an alkaline extraction liquid of rice protein. Therefore, it is important to perform the process described later, i.e. neutralizing rice protein solution, and performing amylase reaction after aggregating rice protein.

As a method of removing alkali component and low-molecular-weight components such as monosaccharides, oligosaccharides, amino acids and peptides from rice protein alkaline extraction liquid from which dietary fiber has been filtrated, causing the rice protein to aggregate by neutralizing the rice protein alkaline extraction liquid and collecting as precipitate, then washing this precipitate to remove remaining low-molecular-weight components can be employed. Although the collection rate of rice protein is the highest in the case of setting the pH after neutralization to 5.0 to 6.0, the interaction between protein molecules changes under the presence of salts, similarly to other vegetable proteins, and it is considered that a stronger, closed aggregate is formed. For that reason, the precipitate of rice protein collected from centrifugation from a neutral liquid containing salts in abundance is packed together and dehydrated in a state in which proteins are strongly intertwined, which is considered to be the cause of the obtained rice-protein composition becoming a hard aggregate for which dispersion is difficult. On the other hand, if utilizing a microfiltration membrane or ultrafiltration membrane in the removal of low-molecular components, protein molecules exist in a state floating in water while loosely aggregating, and low-molecular components are removed by membrane filtration; therefore, the degree of aggregation of the protein obtained ultimately is low, and it is possible to prepare a composition having good dispersibility and excelling in tongue feel and melt-in-the-mouth quality.

The pH of the rice-protein alkali extraction liquid is controlled by the concentration of alkali used in extraction; however, it is usually in the range of 11 to 13, and when performing microfiltration or ultrafiltration with the pH thereof as is, there may be insufficient durability due to the material of the membrane filter. In addition, with a pH of 11 or higher, the aggregation between proteins is low, and depending on the type of membrane, dissolved protein may pass through the filtration membrane, causing a decline in protein yield. In addition, with a pH of 9 or lower, there is a risk of the inside of a hollow fiber membrane or spiral membrane being clogged due to aggregation between proteins, and thus is not preferable. For that reason, the pH of the rice-protein extraction liquid upon performing membrane filtration is desirably adjusted to 9 to 11, and preferably 9.5 to 10.5. As membranes applicable to membrane filtration, a microfiltration membrane having a pore size of 0.05 to 0.45 μm and an ultrafiltration membrane having a molecular weight cutoff of 5,000 to 500,000 are suited. Although the material thereof is not of particular concern so long as being a filtration membrane satisfying the above-mentioned conditions, when considering the durability and adhesion of protein to the membrane surface, a microfiltration membrane made of polyvinylidene fluoride (PVDF) or an ultrafilter made of polyacrylonitrile (PAN) or polyethersulfone (PES) are preferable. By processing the rice-protein alkali extraction liquid using these filtration membranes, the removal of low-molecular weight components and concentration of rice protein will be achieved. By adding suitable water and continuing filtration as necessary, it is possible to more completely carry out removal of low molecular weight components. In the case of performing membrane filtration industrially, it is important to perform filtration with a cross-flow system using a hollow fiber membrane, spiral membrane, tubular membrane or the like that can avoid clogging of the filtration membrane face. It is suitable for the pH of the rice-protein solution in the membrane filtration processing to be in the range of 9 to 10. The pH of the solution gradually declines accompanying removal of alkali component. Since there is a risk of clog of the membrane due to aggregation of proteins being caused when performing filtration until becoming a pH of 9 or lower, it is important to end the operation of membrane filtration prior to the pH of the protein solution becoming 9 or lower. It should be noted that rice protein has a structure that changes around 30°C in alkaline solution and adheres to solids on the filtration membrane surface; therefore, the operation of the membrane filtration is desirably performed at a temperature no higher than 30°C, and more preferably no higher than 25°C.

As a method of collecting protein from concentrated/desalinated liquid of protein on which membrane filtration has ended (hereinafter defined as "rice-protein concentrated liquid"), although it is possible to perform spray drying, drum drying, flash drying, freeze drying, etc. on the rice-protein concentrated liquid as is, in order to reduce the moisture amount to be evaporated, it is also possible to collect protein aggregate after adjusting the pH of the rice-protein concentrated liquid to neutral or weakly acidic by way of centrifuging, etc., and then causing to dry. In this case, since the salts contained in the precipitate of protein are in a small quantity, the degree of entanglement between protein molecules lowers by a certain extent; however, the rice-protein precipitate obtained by adjusting the pH to the range of 5 to 6 become strong aggregates. For this reason, the pH upon collecting precipitate is preferably at least 7.0 or no higher than 4.0. In addition, the rice-protein concentrated liquid may be adjusted in pH to at least 7.0 or no higher than 4.0 and dried as is by spray drying, drum drying or the like.

On the other hand, it is also possible to collect rice protein from rice-protein alkaline extraction solution as is. In a case of causing aggregate to form by neutralizing the rice-protein alkali extraction liquid, preferably an extraction liquid from which fibers have been removed with at least a 200-mesh screen, or a case of causing aggregate to form by adding divalent metal ion, rice protein superior in texture with good tongue feel will be obtain when the pH is adjusted to at least 7.0 or no higher than 4.0 thereafter. Furthermore, the temperature at which aggregate of rice protein is made to form is important, and preferably is a temperature of at least 40°C and no higher than 80°C. The rice protein can thereby form very large, soft aggregates, and can be easily collected by filtration using an at least 200-mesh screen, filter paper or filter cloth. In the case of causing an aggregate of rice protein to form by neutralization, although the amount of collectable protein as aggregate will decline when adjusting the temperature to no higher than 25°C and at least pH 7.0 or no higher than pH 4.0, performing neutralization treatment at a temperature of 40°C or higher is advantageous in that protein can be collected at high efficiency even with at least pH 7.0 or no higher than pH 4.0. It is also possible to re-suspend the collected aggregate as necessary, and desalinate by filtrating or sieving a second time. It is also possible to produce gel-form foods of firm tofu type by storing the aggregate of rice protein in the paste form thus obtained in a mold frame, and leaving for a short while, and a solid protein composition can be collected by allowing to dry after dehydrating the aggregate.

It is also possible to further advance gelation of the protein by further heating the aggregate of rice protein. In order to cause the rice protein to efficiently gel, it is important to set the pH of a solution containing rice protein to at least 11.3, and preferably at least 11.5, adjust to a predetermined pH after causing rice protein aggregate to form by neutralizing this, or aggregate rice protein by adding divalent metal ion, and subsequently heat the suspension containing this aggregate to at least 80°C. The aggregate of gelled protein obtained in this way is a large, soft aggregate almost the same even if the temperature at which first causing to form aggregate is no higher than 40°C or in the range of 40°C to 80°C, and thus there is an advantage also in the aspect of protein being able to be collected at high efficiency even with at least pH 7.0 or no higher than 4.0.

The protein present in the tissue of rice forms protein body, and exists in a state in which protein molecules are firmly folded into layers. In the case of extracting this with alkali, the protein molecules enter a somewhat relaxed state, and the structure of the protein molecules further relaxes when further heating this to at least 40°C and no higher than 80°C, and the free SH groups existing inside of the folded protein molecules is considered to be exposed at the molecule surface. Protein molecules for which the structure has relaxed are folded again by neutralizing. In the range of pH 5 to 6 near the isoelectric point of rice protein, the rice-protein molecules are firmly folded again, and aggregate in a dense state. When heating this to at least 80°C, a part forms a network by SS bonds in a state in which the structure relaxed. At this time, SS bonds are formed in a state in which the protein molecules further relax with at least pH 7.0 or no higher than 4.0, and the network is formed while retaining high porosity. For this reason, after adjusting the pH of protein aggregate to at least 7.0 or no higher than 4.0, it is preferable to cause to gel by heating to at least 80°C. It should be noted that a rice-protein sample gelled by such heat treatment exhibits high water-retaining capacity. It should be noted that it is desirable for heat treatment to be performed immediately after causing the protein to aggregate by neutralization, and even in the case of leaving the solution containing aggregate of rice protein to stand for a long time in the range of pH 4 to 7, or suspending the rice-protein aggregate in water and heated once dried, a rise in water-retaining capacity by gelation sufficiently does not occur. It is conversely disadvantageous in the aspect of causing a decline in texture due to thermal denaturation of protein.

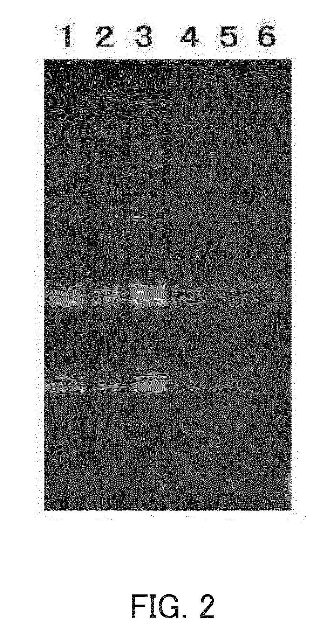

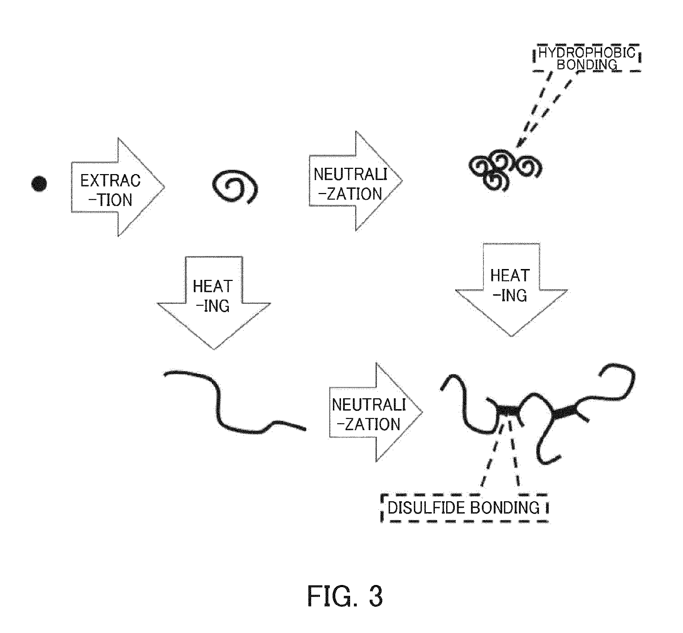

As an experiment to support the model of such molecular structure change, the behavior of free SH groups in the protein molecules could be investigated by fluorescent labeling the protein compositions obtained in the case of neutralizing the alkaline extraction liquid of rice protein at 25°C, the case of neutralizing at 50°C, and the case of heating to 50°C, followed by cooling to 25°C and neutralizing, with monobromobimane, which specifically binds to free SH groups, and subsequently performing SDS polyacrylamide gel electrophoresis (SDS-PAGE) (FIG. 2). In a case of performing neutralization at 25°C (lane 1), or a case of cooling to 25°C after 50°C heating, and then neutralizing (lane 3), a comparatively large amount of SH groups exist in a free state; whereas, in the case of neutralizing at 50°C (lane 2), it is found that the ratio of free SH groups declines dramatically. On the other hand, in the case of heating these after neutralization for 30 minutes at 80°C, almost no free SH groups were recognized. As mechanisms of such aggregation or gelation, the molecular model shown in FIG. 3 is assumed.

The rice protein aggregate produced by forming a network with the porosity remaining high and the rice-protein composition produced by drying this can contain a large amount of water in void portions, and thus it is possible to maintain a fixed shape as a gelatinous composition. The dispersibility and tongue feel of such a composition with high water absorbency improves remarkably compared to a composition of low water absorbency, and is assumed to exhibit good food texture thereby. It should be noted that, in the case of dried rice-protein composition, from centrifugation being performed for 3 minutes at 1,500 x g at room temperature (around 25°C, specifically 25°C), for example, after adding an excess amount of water to the rice-protein composition for which the dry weight is already known so as to sufficiently swell the protein, and the wet weight of the gelatinous composition obtained as a precipitate being measured, and in the case of a gelatinous rice-protein composition, the wet weight of precipitate after removing excess moisture by performing centrifugation for 3 minutes at 1,500 x g and the dry weight from drying this are obtained, whereby the measurement of water-retaining capacity is obtained through formula 1. The rice-protein composition in the embodiment of the present invention has a water-retaining capacity of at least 6 times, and preferably at least 10 times.

$$\text{Water-retaining capacity} = (\text{``wet weight''} - \text{``dry weight''})/\text{``dry weight''}$$

Formula 1

The pH of liquid is substantially equal to the pH of the concentrated liquid prior to drying, in the case of dissolving or suspending the rice-protein composition obtained by drying in water again. The rice-protein composition collected in this way contains glutelin and prolamine, which are the main proteins of rice, in substantially the same proportions as the compositional ratio in rice endosperm. In addition, irrespective of the collection method of rice-protein composition, for example, the pH of the rice protein concentrated liquid or the presence of a step to collect precipitate, the compositional ratio of glutelin and prolamine will not change greatly. It should be noted that the matter of rice-protein composition containing glutelin and prolamine is confirmed by molecular weight fractionating the rice-protein composition with the normal method of SDS-PAGE, and the existence of bands corresponding to glutelin (acidic subunits of molecular weight 37 to 39 kDa and basic subunits of molecular weight 21 to 23 kDa) and prolamine (molecular weight of 10 to 16 kDa).

The dried rice protein is pulverized as necessary, screening is performed using a 60 to 100 mesh sieve to obtain an even-particle size protein composition. The rice-protein composition obtained in this way has little off-taste/odor, excels in water absorbency and dispersibility in water, and has very good tongue feel and a melt-in-the-mouth quality; therefore, it is possible to add to food in a wide range of fields. The foods, for example, may be rice crackers, Japanese confectionery, western confectionery, ice creams, flavorings, meat processed foods, fish and marine processed foods, dairy and egg processed foods, vegetable processed foods, fruit and nut processed foods, and grains processes foods. Thereamong, Japanese and Western confectioneries and boiled fish-paste products are preferably in the aspect of the superiority in excellent tongue feel and a melt-in-the-mouth quality being exhibited.

The rice protein concentrated liquid subjected to dealkalization treatment by membrane filtration can be made to form a gel similarly to bean curd, by adding glucono-delta-lactone (GDL) used when producing bean curd, and then heating. GDL that has been dissolved in water is gradually hydrolyzed to become gluconic acid, whereby the pH of the solution is made to decline. Although it is possible to adjust the pH of the gel formed depending on the added amount of GDL, in the case of the pH of the gel being at least 7.0 or no higher than 4.0, a comparatively soft gel will be formed, which will exhibit very superior food texture in good tongue feel and a melt-in-the-mouth quality that is smooth resembling silken tofu.

EXAMPLES

The protein content of rice-protein compositions was obtained by multiplying 5.95, which is the nitrogen coefficient of rice protein, by the nitrogen content obtained by the oxygen recirculation combustion TCD gas chromatography detection method using a SUMIGRAPH NC-900 manufactured by Sumika Chemical Analysis Service, Ltd. The moisture content of the rice-protein composition was obtained by the weight method by drying for 2 hours at 130°C. In addition, for the matter of the rice-protein composition containing glutelin and prolamine, the rice-protein composition was molecular weight fractionated by SDS-PAGE, and confirmation of bands indicating their presence was carried out.

Example 1

In 1 L of a 0.2% sodium hydroxide aqueous solution, 200 g of Koshihikari rice flour was suspended, and the protein was extracted overnight at room temperature. Starch was removed by centrifuging over 10 minutes at 1,500 x g, and the supernatant was further centrifuged at the same conditions to collect the recentrifuged supernatant thereof. The recentrifuged supernatant (rice protein alkaline extraction liquid) was equally divided in two, one remaining as is, and the other being treated with a 200 mesh sieve (75 $\mu$m mesh size), followed by adjusting the pH of the respective solutions under a room temperature environment (25+/-2°) to 6.0 by 6 N hydrochloric acid. The precipitate was collected by centrifugation (2,000 x g, 10 min), and this was suspended in 200 mL of distilled water. The suspension was centrifuged at the above conditions, and the obtained precipitate was dried by freeze drying. Compared to a rice-protein composition (Sample A) prepared without passing the sieve processing, the composition (Sample B) prepared after passing the sieve processing excelled in grindability and dispersibility, and the tongue feel improved.

Example 2

A rice protein alkaline extraction liquid prepared by the same technique as Example 1 was processed with a sieve of 280 mesh (53 $\mu$m mesh size). Thereafter, the rice-protein composition (Sample C) was collected by the same technique as Example 1.

Example 3

After washing 200 g of Koshihikari white rice, it was immersed over night at room temperature in 1 L of a 0.2% sodium hydroxide aqueous solution. The immersed rice was finely pulverized with a mortar, and then mixed with immersion liquid, agitated with a stirrer for 2 hours, and the protein was extracted. After removing coarse pulverized matter with a sieve of 150 mesh (106 $\mu$m mesh size), centrifugation was performed twice similarly to Example 1 to obtain the rice protein alkaline extraction liquid. After equally dividing this liquid in two, one remaining as is, the other one was subjected to 280 mesh sieve processing, followed by neutralizing at room temperature similarly to Example 1, and rice-protein compositions were obtained by subsequently performing centrifugation and drying (former being Sample D, latter being Sample E).

Example 4

A rice protein alkaline extraction liquid was obtained by the same technique as Example 3. This liquid was sieve processed with 280 mesh, 6 N hydrochloric acid was added to 500 mL of this liquid to adjust the pH to 10.0, followed by concentrating and desalinating until the liquid amount became 100 mL using a pencil-type module USP-043 (microfiltration membrane made of polyvinylidene fluoride) manufactured by Asahi Kasei Corp. To the concentrated liquid, 100 mL of distilled water was added and filtration was continued until the liquid amount became 100 mL, and this operation was repeated four times. To the concentrated liquid, 6 N hydrochloric acid was added to adjust the pH to 5.5, followed by performing centrifugation, and a rice-protein composition was obtained by freeze drying the generated precipitate (Sample F).

Comparison of Rice Protein Samples

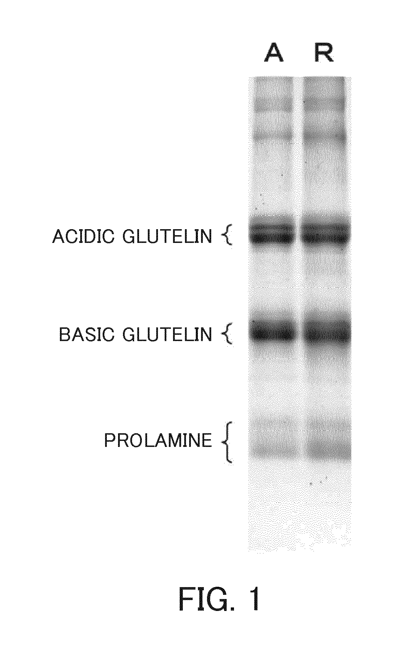

Protein content, grindability, dispersibility and tongue feel were evaluated for Samples A to F. It should be noted that grindability qualitatively evaluated the ease of collapse upon crushing the freeze-dried product with a fingertip, and dispersibility the uniformity of a suspension upon suspending 1 g of the rice protein sample in 10 mL of distilled water. The tongue feel was indicated by the average value of 6-stage sensory rating results of 0 to 5 (higher being superior) by a panel of five people. It should be noted that the moisture contents of these samples were all about 1%. In addition, the results of SDS-PAGE of Sample A are shown in FIG. 1. In addition, although not shown in the drawings, glutelin and prolamine were also contained in Samples B to F similarly to Sample A.

[Table 1]

|  | Protein content (%) | Grindability | Dispersibility | Tongue feel |
|---|---|---|---|---|
| A | 87.9 | × | × | 1.2 |
| B | 93.2 | Δ | Δ | 2.8 |
| C | 94.4 | ○ | ○ | 3.0 |
| D | 89.6 | × | Δ | 2.4 |
| E | 94.7 | ◎ | ○ | 3.6 |
| F | 94.2 | ◎ | ◎ | 4.0 |

As shown in Table 1, it is found that the protein contents relative to solid content of Samples B, C, E and F collected by processing the rice protein alkaline extraction liquid with a sieve of fine sieve size were high, and the grindability, dispersibility and tongue feel were greatly improved. In addition, compared to Sample C extracted from rice flour, Sample E extracted from polished rice had somewhat higher protein content relative to solid content, and the quality rating results were superior, although only slightly. For Sample F subjected to concentrating and desalinating using a microfiltration membrane, although the protein content relative to solid content slightly declined compared to Sample E, it was superior in dispersibility and tongue feel.

Example 5

To 5 L of 0.1% sodium hydroxide aqueous solution, 1 kg of Koshihikari polished rice was added and immersed over night at room temperature. The immersed rice was pulverized with a super mass collider manufactured by MASUKO SANGYO CO., LTD., the pulverization liquid was centrifuged twice, and about 3L of the rice protein alkaline extraction liquid was collected. After adjusting the pH of this liquid to 10.0, it was processed with a 280 mesh sieve. With a pencil-type module ALP-0013 (ultrafilter membrane made from polyacrylonitrile) manufactured by Asahi Kasei Corp., 1 L of the processed liquid was concentrated until 200 mL, 200 mL of distilled water was subsequently added, and the operation of concentrating until the liquid amount became 200 mL was performed a total of 5 times. This liquid was equally divided into ten parts of 20 mL each in 50-mL centrifuge tubes, 6 N hydrochloric acid was added to each to adjust so as to make the pHs shown in Table 2. Centrifugation was performed for 10 minutes at 2,000 x g, the supernatant was discarded, and the precipitate was freeze dried as is. The weight of the obtained precipitate, protein content relative to solid content, and sensory rating by a panel of five people were performed. As shown in Table 2, in the case of the pH after neutralization being at least 7.0 and the case of being no higher than 4.0, the sensory rating scores for tongue feel rose, and all members of the panel evaluated as 5 points for Samples G and H in particular. It should be noted that, although not illustrated, glutelin and prolamine were contained in these samples.

[Table 2]

| Sample | pH | Protein content (%) | Recovered amount (g) | Tongue feel |
|---|---|---|---|---|
| G | 7.5 | 95.1 | 0.78 | 5.0 |
| H | 7.0 | 95.0 | 0.95 | 5.0 |
| I | 6.8 | 94.7 | 1.02 | 4.4 |
| J | 6.5 | 94.7 | 1.12 | 4.0 |
| K | 6.0 | 95.0 | 1.13 | 4.0 |

(continued)

| Sample | pH | Protein content (%) | Recovered amount (g) | Tongue feel |
|--------|-----|---------------------|----------------------|-------------|
| L | 5.5 | 94.8 | 1.12 | 3.8 |
| M | 5.0 | 93.2 | 1.14 | 3.8 |
| N | 4.5 | 92.2 | 1.06 | 3.8 |
| O | 4.0 | 92.1 | 1.01 | 4.8 |
| P | 3.5 | 89.9 | 0.85 | 4.8 |

Example 6

With a 280 mesh sieve, 1 L of the rice protein alkaline extraction liquid obtained in Example 5 was processed. After adjusting 500 mL among the filtrate thereof to a pH of 10.0, it was concentrated with a pencil-type module AHP-0013 (ultrafilter membrane made from polyacrylonitrile) manufactured by Asahi Kasei Corp., until the liquid amount became 100 mL. Addition of 100 mL of distilled water and the concentration process until becoming a liquid amount of 100 mL was repeated five times, and subsequently the precipitate generated by adjusting to a pH of 7.0 was collected by centrifugation (2,000 x g, 10 min). The precipitate was freeze dried, and 4.4 g of rice-protein composition (Sample Q) was collected. The centrifugation supernatant was mixed with 400 mL of the sieve processed rice protein alkaline extraction liquid, and ultrafiltration, pH adjustment, centrifugation and freeze drying were all performed by the same means to obtain 4.6 g of rice-protein composition (Sample R). The protein contents relative to solid content of Sample Q and Sample R were 95.2% and 95.1%, respectively, and the moisture content was about 1% for both. In addition, although the results of SDS-PAGE of Sample R are shown in FIG. 1 as an example, the same SDS-PAGE pattern as Sample A is exhibited also for Sample Q and Sample R, and glutelin and prolamine were contained in almost the same compositional ratios in these samples. As a result of performing sensory rating for these, all members of the panel evaluated tongue feel as 5 points.

Example 7

Rice protein was extracted by suspending 500 g of Koshihikari rice flour in 2 L of a 0.2% NaOH aqueous solution overnight. After performing a centrifugation operation of 10 minutes at 1,500 x g twice, 280 mesh (53 $\mu$m sieve size) sieve processing was performed to obtain a rice protein alkaline extraction liquid. This was dispensed in 80 mL in each of six beakers, and after adding 6 N HCl to each to adjust the pH to 9.0, 8.0, 7.0, 6.0, 5.0 and 4.0, they were equally divided in two, one remaining as is, and the other heated for 5 minutes in boiling water. After centrifuging each for 3 minutes at 1,500 x g to collect aggregate, these were suspended in 40 mL of distilled water, and centrifugation was performed at the above conditions to obtain wet pellets. The weight of wet pellets was measured after carefully removing the supernatant portion and moisture clinging to the inner walls of the centrifuge tube using a mechanical pipette, the dry weight was measured after drying each by freeze drying, and the water-retaining capacity of each sample was obtained by calculating following the aforementioned formula 1.

In addition, after dispensing the rice protein alkaline extraction liquid in 80 mL into each of six beakers similarly, it was heated to 50°C and then the pH was adjusted to 9.0 to 4.0 similarly as described above using 6 N NaCl, aggregate collection by centrifugation and the purification operation with distilled water were performed to obtain dry pellets similarly. Among the 24 samples obtained, grindability was evaluated in 5 stages for 20 samples excluding 4 samples for which the collected amount was very low.

[Table 3]

| Neutralization temperature | pH | No heating by boiling water | | | Heating for 5 minutes in boiling water | | |
|---|---|---|---|---|---|---|---|
| | | Yield (mg) | Water-retaining capacity | Grindability | Yield (mg) | Water-retaining capacity | Grindability |
| Room temperature | 9.0 | 10 | 12.9 | - | 10 | 9.0 | - |
| | 8.0 | 70 | 10.7 | 5 | 70 | 9.3 | 5 |
| | 7.0 | 400 | 4.7 | 4 | 370 | 8.1 | 5 |
| | 6.0 | 450 | 3.9 | 2 | 400 | 7.1 | 4 |
| | 5.0 | 410 | 4.4 | 3 | 390 | 7.0 | 4 |
| | 4.0 | 400 | 13.0 | 5 | 230 | 12.1 | 5 |
| 50°C | 9.0 | 10 | 10.6 | - | 10 | 8.2 | - |
| | 8.0 | 60 | 10.6 | 4 | 60 | 9.0 | 4 |
| | 7.0 | 360 | 6.0 | 4 | 370 | 6.8 | 4 |
| | 6.0 | 380 | 5.1 | 3 | 400 | 6.3 | 3 |
| | 5.0 | 380 | 5.5 | 3 | 390 | 6.7 | 3 |
| | 4.0 | 350 | 10.0 | 4 | 310 | 10.8 | 4 |

As shown in Table 3, samples obtained by neutralizing to a pH of at least 7.0 or no higher than 4.0 were superior in grindability. On the other hand, samples obtained by neutralizing the rice protein alkaline extraction liquid to a pH of 6.0 or 5.0 at room temperature had extremely inferior grindability. The water-retaining capacity of the samples obtained by neutralizing at 50°C was generally high compared to cases of neutralizing at room temperature, and there was a trend of the water-retaining capacity further rising by performing heat treatment in boiling water after neutralization.

Example 8

To each of 40 mL of 0.025% to 0.2% NaOH aqueous solutions, 10 g of Koshihikari rice flour was added and suspended, and protein was extracted over night at room temperature. The supernatant was collected by centrifugation, and after neutralizing this to a pH of 7.0, it was heated for 5 minutes in boiling water. The precipitate collected by centrifugation for 3 minutes at 1,500 x g was suspended in 40 mL of distilled water, and centrifugation was performed at the same conditions to obtain wet pellets of protein. The yield and water-retaining capacity were obtained by measuring the wet weight, and measuring the dry weight after freeze drying similarly to Example 7.

[Table 4]

| NaOH (%) | Immersion liquid pH | Neutralization pH | Yield (mg) | Water-retaining capacity |
|---|---|---|---|---|
| 0.025 | 9.5 | 7.0 | 0.1 | - |
| 0.05 | 10.7 | 7.0 | 8 | - |
| 0.075 | 11.1 | 7.0 | 251 | 5.8 |
| 0.1 | 11.3 | 7.0 | 268 | 6.4 |
| 0.125 | 11.6 | 7.0 | 309 | 7.2 |
| 0.15 | 11.7 | 7.0 | 298 | 7.9 |
| 0.175 | 11.8 | 7.0 | 309 | 6.3 |
| 0.2 | 11.9 | 7.0 | 333 | 7.1 |

As in the results shown in Table 4, at a NaOH concentration during immersion of at least 0.1%, the water-retaining capacity of gel was at least 6.

Example 9

Rice protein was extracted using 0.025 to 0.2% NaOH aqueous solutions exactly the same as Example 8, and after neutralizing these to a pH of 5.5, were heated for 5 minutes in boiling water. Centrifugation and purification were performed in the same way, and the wet weight and dry weight after freeze drying were obtained. Although results are shown in Table 5, at the NaOH concentration during immersion of at least 0.1% similarly to the case of Example 8, a gel having a water-retaining capacity of at least 6 was obtained. According to the above results, it is consider important in order to form a gel having high water-retaining capacity to form a gel by heating the protein aggregate obtained by neutralizing a protein solution of at least a pH of 11.3, and preferably at least a pH of 11.5.

[Table 5]

| NaOH (%) | Immersion liquid pH | Neutralization pH | Yield (mg) | Water-retaining capacity |
|----------|--------------------|--------------------|------------|--------------------------|
| 0.025 | 9.6 | 5.5 | 54 | 7.1 |
| 0.05 | 10.6 | 5.5 | 165 | 6.0 |
| 0.075 | 11.0 | 5.5 | 283 | 5.9 |
| 0.1 | 11.3 | 5.5 | 318 | 7.0 |
| 0.125 | 11.5 | 5.5 | 340 | 7.8 |
| 0.15 | 11.6 | 5.5 | 352 | 7.9 |
| 0.175 | 11.7 | 5.5 | 368 | 8.2 |
| 0.2 | 11.8 | 5.5 | 368 | 7.8 |

Example 10

By the same method as Example 5, 4 L of rice protein extraction liquid was obtained. After performing 280 mesh sieve processing, the pH was adjusted to 10.0. Using a laboratory module AHP-1013 (ultrafiltration membrane made from polyacrylonitrile) manufactured by Asahi Kasei Corp., an operation of concentrating until the liquid amount became 1 L, adding 3 L of distilled water to this, and further concentrating until the liquid amount became 1L was repeated twice. The pH of the obtained rice protein concentrated liquid was 9.1. This liquid was equally divided into each of four portions with an amount of about 250 mL, and 6 N HCl was added to each liquid to adjust the pH to 9.0, 8.0, 7.5 and 7.0. Each liquid was dispensed in 50 mL into each of four 100 mL tall beakers, a 10% glucono-delta-lactone aqueous solution was added immediately after preparation so that the final concentration of glucono-delta-lactone of each became 0.1, 0.2, 0.5 and 1.0%, and then placed in boiling water and heated for 15 minutes. The firmness of the generated gel was qualitatively evaluated in the three stages of + (soft) to +++ (firm). In addition, the pH of the gel was measured by directly inserting pH electrodes into the gel. The results are shown in Table 6. It should be noted that a gel for which the gel strength is displayed as - indicates not having gelled.

[Table 6]

| Pre-reaction pH | | Glucono-delta-lactone (%) | | | |
|-----------------|--------------------------|------|------|------|------|
| | | 0.1 | 0.2 | 0.5 | 1.0 |
| 9.0 | Degree of saponification | - | +++ | ++ | + |
| | Gel pH | 7.40 | 6.17 | 4.34 | 3.86 |
| 8.0 | Degree of saponification | + | +++ | ++ | + |
| | Gel pH | 7.01 | 5.94 | 4.27 | 3.84 |
| 7.5 | Degree of saponification | ++ | +++ | ++ | + |
| | Gel pH | 6.63 | 5.44 | 4.23 | 3.83 |
| 7.0 | Degree of saponification | +++ | +++ | ++ | + |
| | Gel pH | 6.09 | 5.03 | 4.13 | 3.78 |

Except for one sample which did not form a gel, samples having a pH of the gel of at least 7.0 or no higher than 4.0 were soft gels judged as +.

Example 11

By performing the same processing as Example 10, about 1 L of rice protein concentrated liquid was obtained. The pH of the concentrated liquid was 9.2. A 10% aqueous solution of glucono-delta-lactone was added to this so that the final concentration became 0.2%, and was filled into a 200-mL plastic container. After sealing by covering, a gel was formed by heating in boiled water for 15 minutes. The pH of the gel obtained was 7.2, and exhibited a soft, smooth texture similar to silken tofu. The gel was almost tasteless and odorless; however, a faint rice flavor could be sensed.

**Claims**

1. A rice-protein composition comprising glutelin and prolamine, wherein the protein content with respect to the solid content is at least 90%, and the composition can retain water in at least 6 times its weight while maintaining a gelatinous form.

2. A rice-protein composition comprising glutelin and prolamine, wherein a pH of liquid when dissolved or suspended in water is at least 7.0 or no higher than 4.0.

3. A method for manufacturing a rice-protein composition, comprising the steps of: forming an aggregate of protein by neutralizing a solution containing rice protein, or by adding a divalent metal ion to the solution; and gelling to collect the aggregate by heating to at least 80°C.

4. The method according to claim 3, wherein the pH of a solution containing rice protein is at least 11.3.

5. The method according to claim 3, wherein the generation of the aggregate is performed at a temperature of at least 40°C and no higher than 80°C.

6. The method according to any one of claims 3 to 5, wherein the pH of the solution during the heating is set to at least 7.0 or no higher than 4.0.

7. The method according to any one of claims 3 to 6, comprising a step of passing a solution containing rice protein through a sieve of no more than 75 $\mu$m mesh size.

8. The method according to any one of claims 3 to 7, comprising a step of concentrating and desalinating rice protein by membrane filtrating an alkaline extraction liquid containing rice protein using a microfiltration membrane or ultrafiltration membrane.

9. A method for manufacturing a rice-protein composition comprising a step of adjusting a solution containing rice protein to a pH of at least 7.0 or no higher than 4.0, and then drying as is, or collecting a precipitate formed and then drying.

10. A method for manufacturing a rice-protein composition comprising a step of adding glucono-delta-lactone to a solution containing rice protein in an amount making a final pH of a gel at least 7.0 or no higher than 4.0, and then causing protein to gel by heating to at least 80°C.

FIG. 1

FIG. 2

FIG. 3

**INTERNATIONAL SEARCH REPORT**

| International application No. |
| --- |
| PCT/JP2012/078660 |

A. CLASSIFICATION OF SUBJECT MATTER
*A23J3/14*(2006.01)i

According to International Patent Classification (IPC) or to both national classification and IPC

B. FIELDS SEARCHED

Minimum documentation searched (classification system followed by classification symbols)
A23J3/14

Documentation searched other than minimum documentation to the extent that such documents are included in the fields searched

| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)
CA(STN), JSTPlus/JMEDPlus/JST7580(JDreamII), WPI

C. DOCUMENTS CONSIDERED TO BE RELEVANT

| Category* | Citation of document, with indication, where appropriate, of the relevant passages | Relevant to claim No. |
| --- | --- | --- |
| X/A | CN 101869173 A (Changsha Xiangbo Medical Technology Co., Ltd.), 27 October 2010 (27.10.2010), paragraph [0016]; examples 1 to 3 (Family: none) | 1-6,9/7,8,10 |
| X/A | JP 2009-519906 A (DSM IP Assets B.V.), 21 May 2009 (21.05.2009), example 1-1 & US 2009/0258954 A1 & EP 1971218 A1 & WO 2007/065718 A1 & KR 10-2008-0075029 A & CN 101600357 A | 1,2/3-10 |

☒ Further documents are listed in the continuation of Box C. ☐ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| 28 January, 2013 (28.01.13) | 05 February, 2013 (05.02.13) |

| Name and mailing address of the ISA/<br>Japanese Patent Office | Authorized officer |
| --- | --- |
| Facsimile No. | Telephone No. |

Form PCT/ISA/210 (second sheet) (July 2009)

**INTERNATIONAL SEARCH REPORT**

| International application No. |
|---|
| PCT/JP2012/078660 |

C (Continuation). DOCUMENTS CONSIDERED TO BE RELEVANT

| Category* | Citation of document, with indication, where appropriate, of the relevant passages | Relevant to claim No. |
|---|---|---|
| A | JP 2006-273840 A (Kameda Seika Co., Ltd. et al.), 12 October 2006 (12.10.2006), entire text (Family: none) | 1-10 |
| A | PARAMAN, I. et al., Preparation of Rice Endosperm Protein Isolate by Alkali Extraction., Cereal Chem., 2008, Vol.85 No.1, pages76-81 | 1-10 |
| A | JP 2007-068454 A (Kizakura Corp.), 22 March 2007 (22.03.2007), example 3 (Family: none) | 10 |
| A | JP 06-062778 A (Minoru IKENO), 08 March 1994 (08.03.1994), paragraph [0002] (Family: none) | 10 |
| A | JP 08-154613 A (Kao Corp.), 18 June 1996 (18.06.1996), paragraph [0022] (Family: none) | 10 |
| A | JP 2002-027935 A (Kyoko KAWANO et al.), 29 January 2002 (29.01.2002), paragraph [0012] (Family: none) | 10 |
| P,A | JP 2012-016325 A (Kameda Seika Co., Ltd. et al.), 26 January 2012 (26.01.2012), entire text (Family: none) | 1-10 |
| P,A | JP 2012-016326 A (Kameda Seika Co., Ltd. et al.), 26 January 2012 (26.01.2012), entire text (Family: none) | 1-10 |

Form PCT/ISA/210 (continuation of second sheet) (July 2009)

## INTERNATIONAL SEARCH REPORT

| International application No. |
| --- |
| PCT/JP2012/078660 |

**Box No. II      Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:
      The technical feature common to the invention of claim 1 and the invention of claim 2 is a rice protein composition which contains a glutelin and a prolamin.
      The technical feature common to the invention of claim 1, the invention of claims 3-8, the invention of claim 9 and the invention of claim 10 is being related to a rice protein composition.
(Continued to extra sheet)

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**      ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**INTERNATIONAL SEARCH REPORT**

International application No.

PCT/JP2012/078660

Continuation of Box No.III of continuation of first sheet(2)

However, the above-said technical features cannot be considered to be a special technical feature, since the technical features do not make a contribution over the prior art in the light of the contents disclosed in the document 2 (JP 2009-519906 A (DSM IP Assets B.V.), 21 May 2009 (21.05.2009), example 1-1).

Further, there is no other same or corresponding special technical feature between these inventions.

Accordingly, the following five inventions (invention groups) are involved in claims.

(Invention 1) the invention of claim 1

A rice protein composition which contains a glutelin and a prolamin, has a protein content of 90% or more relative to the solid contents, and is capable of holding water in an amount of not less than 6 times the weight thereof, while maintaining the state of a gel.

(Invention 2) the invention of claim 2

A rice protein composition which contains a glutelin and a prolamin and has a pH of not less than 7.0 or not more than 4.0 when dissolved or suspended in water.

(Invention 3) the inventions of claims 3-8

A method for producing a rice protein composition, which comprises a step wherein agglomerates of a protein are formed by neutralizing a solution that contains a rice protein or adding divalent metal ions into the solution, and the agglomerates are heated to 80°C or higher so that they are gelled and collected.

(Invention 4) the invention of claim 9

A method for producing a rice protein composition, which comprises a step wherein a solution that contains a rice protein is regulated to have a pH of not less than 7.0 or not more than 4.0 and then directly dried, or alternatively, then precipitated so that the precipitate is collected and dried.

(Invention 5) the invention of claim 10

A method for producing a rice protein composition, which comprises a step wherein glucono delta lactone is added into a solution that contains a rice protein and the resulting is heated to 80°C or higher so that the protein is gelled, said glucono delta lactone being added in such an amount that the final pH of the resulting gel is not less than 7.0 or not more than 4.0.

Form PCT/ISA/210 (extra sheet) (July 2009)

**EP 2 781 164 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006273840 A
- JP 2010156467 A

- JP 2010156466 A

**Non-patent literature cited in the description**

- **KUMAGAI et al.** *J. Nutr. Sci. Vitaminol.,* 2006, vol. 52, 467-572
- **KUMAGAI et al.** *J. Nutr. Sci. Vitaminol.,* 2009, vol. 55, 170-177

- Starch Science Handbook. Asakura Press, 1977, 385-395